# EUROPÄISCHE PATENTSCHRIFT

(11) **EP 0 896 006 B1**
(45) Veröffentlichungstag und Bekanntmachung des Hinweises auf die Patenterteilung: **03.11.2004**
(21) Anmeldenummer: 98114881.0
(22) Anmeldetag: 07.08.1998
(51) Int. Cl.: C08F 218/10, C08F 216/16, G02B 1/04, A61K 7/42

(54) **Lipophile polymere UV-Absorber**
Lipophilic UV-absorbant polymers
Polymères lipophiles absorbant les rayons UV

(30) Priorität: 08.08.1997 DE 19734445
(43) Veröffentlichungstag der Anmeldung: 10.02.1999
(73) Patentinhaber: BASF AKTIENGESELLSCHAFT, 67056 Ludwigshafen (DE)
(72) Erfinder: Keller, Harald, 67069 Ludwigshafen (DE); Schehlmann, Volker, 67354 Römerberg (DE); Westenfelder, Horst, 67435 Neustadt (DE); Preiss, Thomas, 67065 Ludwigshafen (DE)

(56) Entgegenhaltungen:
- EP-A- 0 123 368
- GB-A- 1 460 649
- US-A- 3 992 356
- DATABASE WPI Week 9145 Derwent Publications Ltd., London, GB; AN 329241 XP002142436 & JP 03 220213 A (DAINIPPON INK & CHEM. CO.)

## Beschreibung

Die vorliegende Erfindung betrifft lipophile polymere UV-Absorber auf der Basis von Benzoesäurederivat-Chromophoren und kosmetische Mittel mit polymergebundenen Benzoesäurederivat-Chromophoren zum Schutz der Haut und der Haare vor UV-Strahlung.

Bei kosmetischen Sonnenschutzmitteln, die auf der Haut aufgetragen werden, ist häufig eine gute Wasserfestigkeit wünschenswert. Diese Eigenschaft wird insbesondere gewünscht, wenn häufiger Kontakt der Haut mit Wasser oder wäßrigen Flüssigkeiten wie Schweiß stattfindet. Um die gewünschte Wasserfestigkeit zu erreichen, werden solchen Mitteln häufig Polymere zugesetzt.

Eine weitere Möglichkeit besteht darin, die UV-absorbierenden Gruppen kovalent mit einem Polymer zu verknüpfen.

In WO 89/4824 werden Copolymere aus dem UV-absorbierenden Styrol und Maleinsäureanhydrid, Vinylpyrrolidon oder Acrylaten beschrieben. Diese Copolymere besitzen eine hohe Wasserlöslichkeit und eine geringe Hautpenetration.

In JP 60/88066 werden UV-absorbierende Benzophenonderivate beschrieben, die über einen Urethanspacer an (Meth)acrylate gebunden sind, beschrieben. Diese Polymere eignen sich als Material für Kontaktlinsen und Brillengläser.

In EP 123 368 werden polymere Sonnenschutzmittel beschrieben, die aus folgenden Monomeren aufgebaut sind:
(a) einem olefinischen p-Aminobenzoesäureester,
(b) N-Vinylpyrrolidon und
(c) einem Vinyllactam, Acrylat oder Methacrylat sowie Mischungen davon.

In JP 03220213 werden polymere UV-Absorber aus:
(a) Vinyl-N,N-dimethylaminobenzoat,
(b) Methylmethacrylat,
(c) Styrol,
(d) Hydroxybutylvinylether,
5 (e) Methacrylsäure und
(f) Divinylbenzol beschrieben.

Neben der guten Wasserfestigkeit sollen die kosmetischen Mittel jedoch eine Reihe von anderen Eigenschaften wie geringe Hautpenetration, geringer Gehalt an Restmonomeren, die aus toxikologischen und olfaktorischen Gründen zu vermeiden sind, gute Verarbeitbarkeit, Mischbarkeit und Stabilität mit weiteren Komponenten kosmetischer Mittel, erfüllen.

Zusätzlich sollen die polymeren UV-Absorber eine hohe Extinktion im UV-Bereich sowie eine gute Öllöslichkeit aufweisen.

Gefunden wurden lipophile polymere UV-Absorber mit den in der schematisierten Formel I gezeigten Einheiten 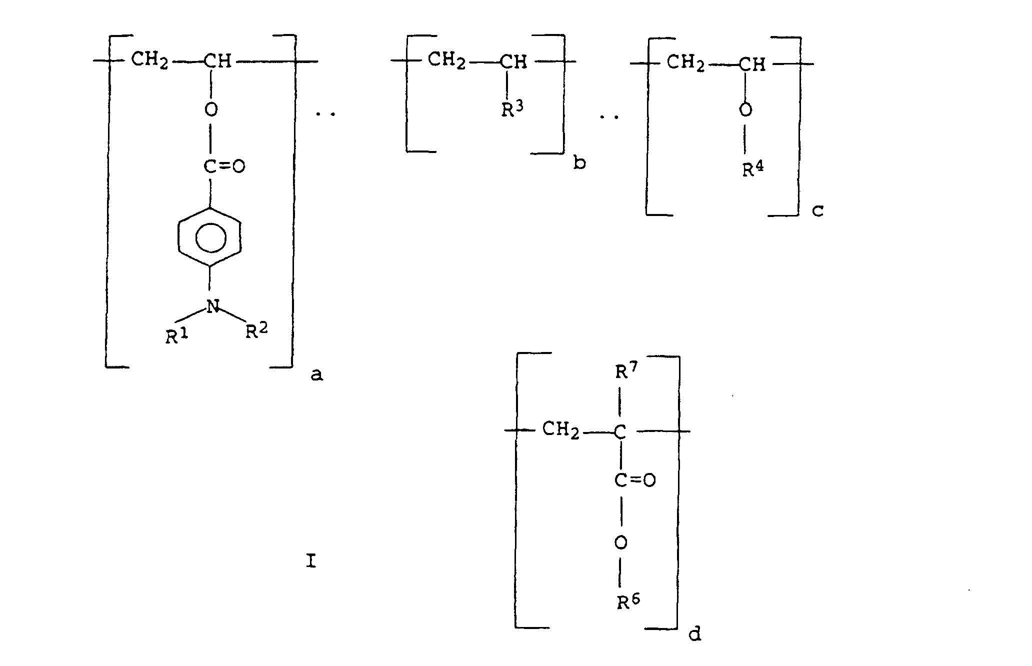 wobei
die Reihenfolge der Einheiten beliebig ist, die Summe aus a+b+c+d = 100 ist und
a für einen Wert von 5 bis 95 steht,
b für einen Wert von 0 bis 70 steht,
c für einen Wert von 5 bis 95 steht,
d für einen Wert von 0 bis 70 steht

- R¹, R²: unabhängig voneinander für H, C₁-C₈-Alkyl
- R³: für 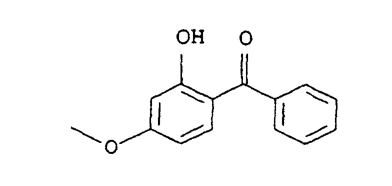 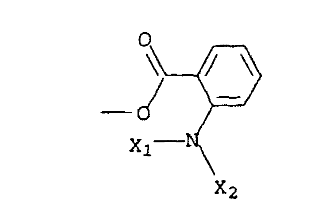
- X¹, X²: voneinander unabhängig für H, C₁ - C₈-Alkyl, 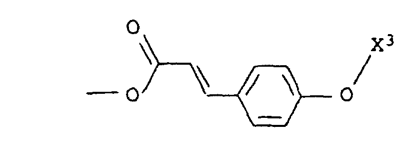
- X³: für H, C₁ - C₈-Alkyl, 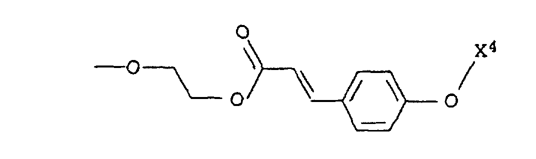
- X⁴: für H, C₁ - C₈-Alkyl, 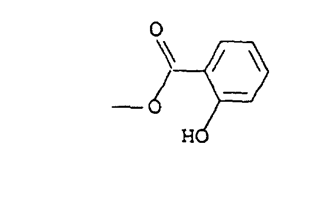 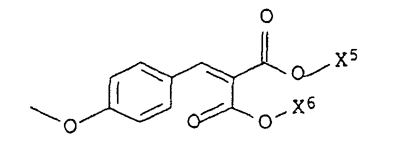
- X⁵, X⁶: voneinander unabhängig für H, C₁ - C₈-Alkyl, 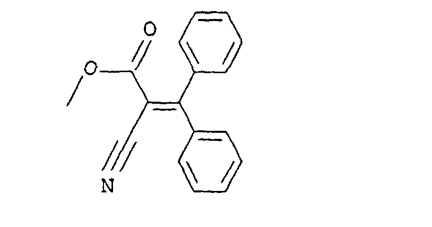 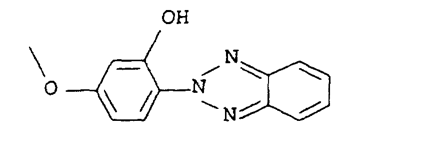 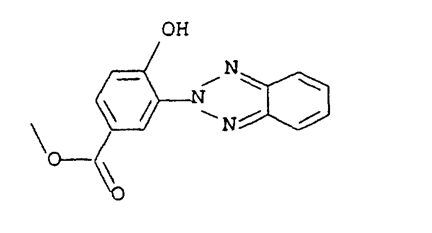 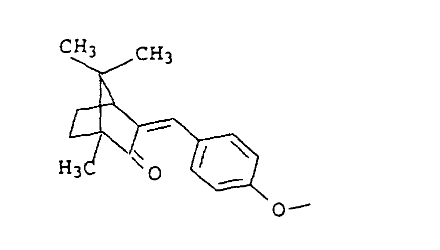 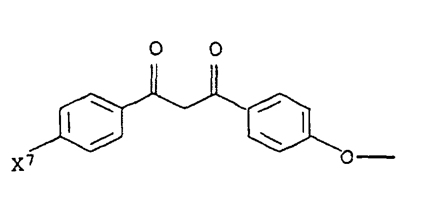
- X⁷: für H, C₁- bis C₈-Alkyl;
- R⁴: -(CO)ₙ-R⁵,
- R⁵: für C₁-C₂₈-Alkyl,
- n: für 0 oder 1,
- R⁶: für C₁-C₂₂-Alkyl,
- R⁷, R⁸: unabhängig voneinander für H oder CH₃
steht.

Bevorzugte Polymere sind solche, bei denen die Monomereinheiten a bis d folgende Werte besitzen:
a 15 - 50
b 5 - 70
c 30 - 80
d 0 - 20

Bevorzugte Monomere, die die Einheit a bilden, sind solche, bei denen R¹ und R² unabhängig voneiander H, CH₃, C₂H₅ bedeutet, besonders bevorzugt steht R¹ und R² für CH₃.

Bevorzugte Monomere, die die Einheit b bilden, sind solche der Formel

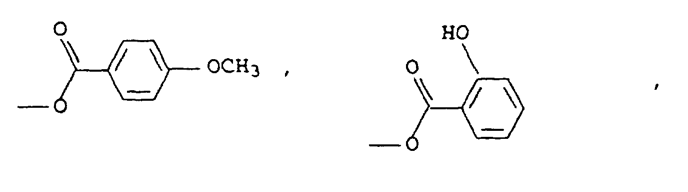

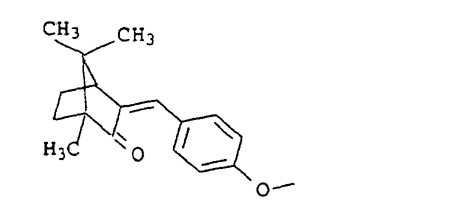

Die Herstellung der Monomere für die Einheit b ist bekannt oder aus analogen Umsetzungen für den Fachmann leicht durchführbar.

Die Herstellung der Monomere der allgemeinen Formel II 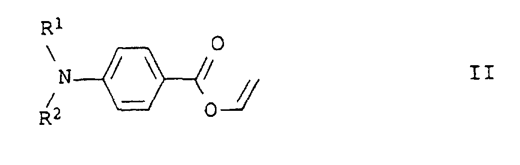 ist in US 661 005, FR 691 020 beschrieben.

Die Herstellung der Monomere der allgemeinen Formel IV 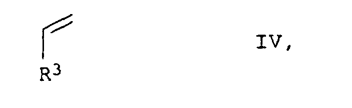 wobei R³ für 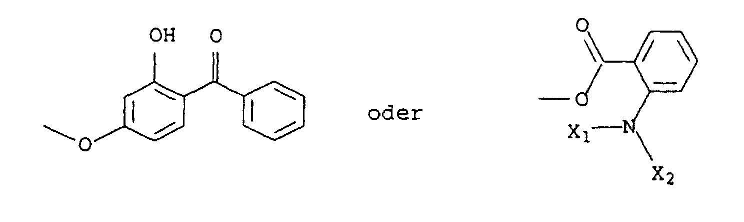 (X₁ = X₂ = H) steht, ist von G. Ciolfi et al., Vitis (1995), 34(3), 195-196 beschrieben.

Für R³ = 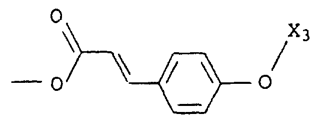 X₃ = CH₃
wird die Herstellung von M. Majeric et al., Tetrahedron: Asymmetry (1996), 7(3), 815-824 beschrieben.

Für R³ = 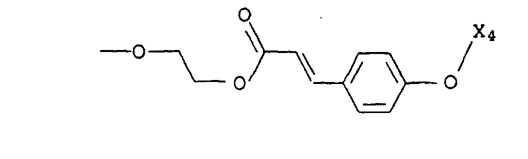 X₄ = CH₃
wird die Herstellung von T.G. Biryukova et al., Vysokomol. Soedin., Ser.B (1978), 20(8), 565-568 beschrieben.

Für R³ = 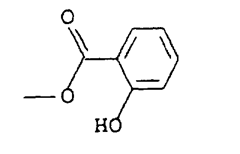 wird die Herstellung in US 5 155 253 und US 921 013 beschrieben.

Allgemein können die chromophortragenden Vinylester durch Umesterung der entsprechenden carboxylgruppenhaltigen UV-Licht absorbierenden Chromophore mit Vinylacetat (siehe G. Heublein, B. Heublein, B. Heyroth, E. Brendel, Z. Chem. 19(1979) 104) erhalten werden.

Allgemein können die chromatophortragenden Vinylether durch Reaktion der OH-Gruppen tragenden UV-Licht absorbierenden Chromophoren mit Acetylen (siehe Organikum, Deutscher Verlag der Wissenschaften, Berlin, 1979, S. 338) erhalten werden.

Unter den Monomeren, die die Einheit c bilden, sind diejenigen mit R⁴ = -CO-R⁹ bevorzugt, wobei R⁹ für ein verzweigtes Alkyl mit 6 bis 12 C-Atomen, insbesondere für 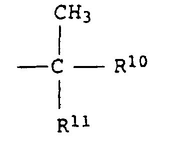 steht und R¹⁰ und R¹¹ zusammen 6 bis 7 C-Atome umfassen, steht.

Unter den Monomeren, die die Einheit d bilden, sind diejenigen mit R⁶ C₄- bis C₁₈-Alkyl, insbesondere tert. -Butyl, 2-Ethylhexyl oder Lauryl bevorzugt.

Die Herstellung der erfindungsgemäßen Polymere erfolgt, indem man die Monomere, die die entsprechende Einheit a - d bilden, unter den üblichen Bedingungen radikalisch polymerisiert.

Als Lösungsmittel eignen sich die üblichen organischen Lösungsmittel, insbesondere Alkohole wie Ethanol, Isopropanol, Butanol, Ester wie Ethylacetat und Butylacetat, Ketone wie Aceton, Methylethylketon und Cyclohexanon, Ether wie Tetrahydrofuran und Methyl-tert.-Butylether und Alkane wie Hexan, Heptan, Cyclohexan und Octan. Vorteilhaft ist auch die Polymerisation in einem kosmetischen Öl wie Paraffinöl, Ester der Benzoesäure mit langkettigen Fettalkoholen, Ester von Glycerin mit langkettigen Fettsäuren, 2-Octyldodecanol, Isostearylalkohol oder Ester der 2-Ethylhexansäure mit langkettigen Fettalkoholen. Besonders bevorzugt ist hierbei ein Ester aus Benzoesäure und C₁₂-C₁₆-Alkoholen. Ferner ist auch die Polymerisation in Wasser möglich, indem die Monomere in Wasser emulgiert werden und mit einem wasserlöslichen Starter polymerisiert werden.

Als Starter eignen sich Azostarter wie z.B. Azoisobutyronitril und Peroxide wie z.B. Dibenzoylperoxid, tertiär-Butyl-2-ethylperhexanoat oder Peroxodisulfate. Die Reaktionstemperatur beträgt 30 bis 160°C, bevorzugt 80 bis 120°C.

Die erfindungsgemäßen Polymere haben in der Regel ein Molekulargewicht von 2000 bis 50 000, bevorzugt von 4000 bis 20 000 g/mol.

Das erhaltene Polymer wird dann mit in der Kosmetik üblichen Hilfs- und Zusatzstoffen je nach Anwendungszweck formuliert. Beispiele geeigneter Hilfs- und Zusatzstoffe für Sonnenschutzmittel sind bei W. Umbach, Kosmetik, Georg-Thieme-Verlag Stuttgart, 1988 beschrieben.

Neben den polymergebundenen UV-Absorbern können die kosmetischen Zusammensetzungen auch weitere, lösliche UV-Absorber enthalten.

Bevorzugt werden solche weiteren UV-Absorber zugemischt, die den Absorptionsbereich des polymergebundenen UV-Absorbers komplementieren, so daß ein möglichst wirksamer Schutz über einen großen UV-Bereich erreicht wird.

Insbesondere erreicht man durch Zugabe einer löslichen UV-A-Absorbers einen guten UV-Schutz in Verbindung mit dem polymergebundenen UV-B-Absorber.

Die erfindungsgemäßen kosmetischen Mittel zeichnen sich durch ihre hohe Wasserbeständigkeit, gute Hautverträglichkeit und geringe Hautpenetration aus. Ferner weisen sie eine gute Hydrolysestabilität, excellente Öllöslichkeit und hohe Extinktion auf.

Die Erfindung wird durch die folgenden Beispiele weiter veranschaulicht.

### Beispiel 1

### Herstellung eines lipophilen UV-Licht absorbierenden Polymers

20 g Butylacetat werden unter Stickstoff vorgelegt und auf 100°C erwärmt. Innerhalb von 2 h werden aus getrennten Gefäßen eine Lösung von 8 g Dimethylaminobenzoesäure-vinylester in 32 g Neononansäurevinylester und eine Lösung von 0,4 g t-Butylperoxy-2-ethylhexanoat in 10 g Butylacetat zudosiert. Nach Beendigung der Zugabe der beiden Lösungen wird 4 h bei 100°C gerührt. Nach dem Erkalten wird die Lösung in 800 ml Ethanol gegossen und das ausfallende Polymer durch Filtration abgetrennt.
Ausbeute: 21 g
Erweichungspunkt: 115 bis 118°C
GPC: Mn = 14 000 g/mol, Polydispersität = 2.30
UV: 310 nm, E¹₁ = 346

### Beispiel 2

### Herstellung eines lipophilen UV-Licht absorbierenden Polymers

20 g Butylacetat werden unter Stickstoff vorgelegt und auf 100°C erwärmt. Innerhalb von 2 h werden aus getrennten Gefäßen eine Lösung von 12 g Dimethylaminobenzoesäure-vinylester in 28 g Neononansäurevinylester in 20 g Butylacetat und eine Lösung von 0,4 g t-Butylperoxy-2-ethylhexanoat in 10 g Butylacetat zudosiert. Nach Beendigung der Zugabe der beiden Lösungen wird 4 h bei 100°C gerührt. Nach dem Erkalten wird die Lösung in 1000 ml Methanol gegossen und das ausfallende Polymer durch Filtration abgetrennt.
Ausbeute: 19,1 g
Erweichungspunkt: 98 bis 102°C
GPC: Mn = 8900 g/mol, Polydispersität = 2.27
UV: 308 nm, E¹₁ = 503

### Beispiel 3

### Herstellung eines lipophilen UV-Licht absorbierenden Polymers

20 g Butylacetat werden unter Stickstoff vorgelegt und auf 100°C erwärmt. Innerhalb von 2 h werden aus getrennten Gefäßen eine Lösung von 16 g Dimethylaminobenzoesäure-vinylester in 24 g Neononansäurevinylester und eine Lösung von 0,4 g t-Butylperoxy-2-ethylhexanoat in 10 g Butylacetat zudosiert. Nach Beendigung der Zugabe der beiden Lösungen wird 4 h bei 100°C gerührt.

Nach dem Erkalten wird die Lösung in 1000 ml Ethanol gegossen und das ausfallende Polymer durch Filtration abgetrennt.
Ausbeute: 10,9 g
Erweichungspunkt: 107 bis 110°C
GPC: Mn = 9400 g/mol, Polydispersität = 1,94
UV: 308 nm, E¹₁ = 606

Allgemeine Herstellvorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

Zubereitungen

### Beispiel 4

Zusammensetzung für die Lippenpflege

| | |
|---|---|
| ad 100 | Eucerinum anhydricum |
| 10,00 | Glycerin |
| 10,00 | Titanium Dioxid |
| 0,5-10 | Polymer aus Beispiel 1 |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 5

Zusammensetzung für die Lippenpflege

| | |
|---|---|
| ad 100 | Eucerinum anhydricum |
| 10,00 | Glycerin |
| 10,00 | Titanium Dioxid |
| 0,5-10 | Polymer aus Beispiel 1 |
| 5,00 | Butyl Methoxydibenzoylmethan |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 6

Zusammensetzung für Sunblocker mit Mikropigmenten

| | |
|---|---|
| ad 100 | Wasser |
| 5,00 | Butyl Methoxydibenzoylmethan |
| 6,00 | PEG-7-Hydrogenated Castor Öl |
| 6,00 | Titanium Dioxid |
| 0,5-10 | Polymer aus Beispiel 3 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 7

Zusammensetzung für Sunblocker mit Mikropigmenten

| | |
|---|---|
| ad 100 | Wasser |
| 10,00 | Octyl Methoxcinnamat |
| 6,00 | PEG-7-Hydrogenated Castor Öl |
| 6,00 | Titanium Dioxid |
| 0,5-10 | Polymer aus Beispiel 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Ö |
| 0,50 | Tocopheryl Acetat |
| 0,50 | Phenoxyethanol |
| 0,20 | EDTA |

### Beispiel 8

### Fettfreies Gel

| | |
|---|---|
| ad 100 | Wasser |
| 8,00 | Octyl Methoxycinnamat |
| 7,00 | Titanium Dioxid |
| 0,5-10 | Polymer aus Beispiel 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 9

### Fettfreies Gel

| | |
|---|---|
| ad 100 | Wasser |
| 8,00 | Octyl Methoxycinnamat |
| 7,00 | Titanium Dioxid |
| 0,5-10 | Polymer aus Beispiel 1 |
| 5,00 | Glycerin |
| 5,00 | PEG-25 PABA |
| 1,00 | Butyl Methoxydibenzoylmethan |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 10

### Sonnencreme

| | |
|---|---|
| ad 100 | Wasser |
| 8,00 | Octyl Methoxycinnamat |
| 8,00 | Titanium Dioxid |
| 6,00 | PEG-7-Hydrogenated Castor Öl |
| 0,5-10 | Polymer aus Beispiel 3 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 11

### Sonnencreme

| | |
|---|---|
| ad 100 | Wasser |
| 8,00 | Octyl Methoxycinnamat |
| 8,00 | Titanium Dioxid |
| 6,00 | PEG-7-Hydrogenated Castor Öl |
| 0,5-10 | Polymer aus Beispiel 2 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 12

### Sonnencreme wasserfest

| | |
|---|---|
| ad 100 | Wasser |
| 4,00 | Butyl Methoxydibenzoylmethan |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 0,5-10 | Polymer aus Beispiel 1 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 13

### Sonnencreme wasserfest

| | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 0,5-10 | Polymer aus Beispiel 2 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 14

### Sonnenmilch

| | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Butyl Methoxydibenzoylmethan |
| 0,5-10 | Polymer aus Beispiel 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0.60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

### Beispiel 15

### Sonnenmilch

| | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 0,5-10 | Polymer aus Beispiel 1 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

### Beispiel 16

### Sonnencreme wasserfest

| | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 0,5-10 | Polymer aus Beispiel 1 |
| 0,5-10 | Polymer aus Beispiel 3 |
| 4,00 | Glycerin |
| 3,00 | Jojoba Öl |
| 2,00 | Butyl Methoxybenzoylmethan |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

## Patentansprüche

1. Polymere UV-Absorber auf Basis der in der schematisierten Formel I gezeigten Einheiten 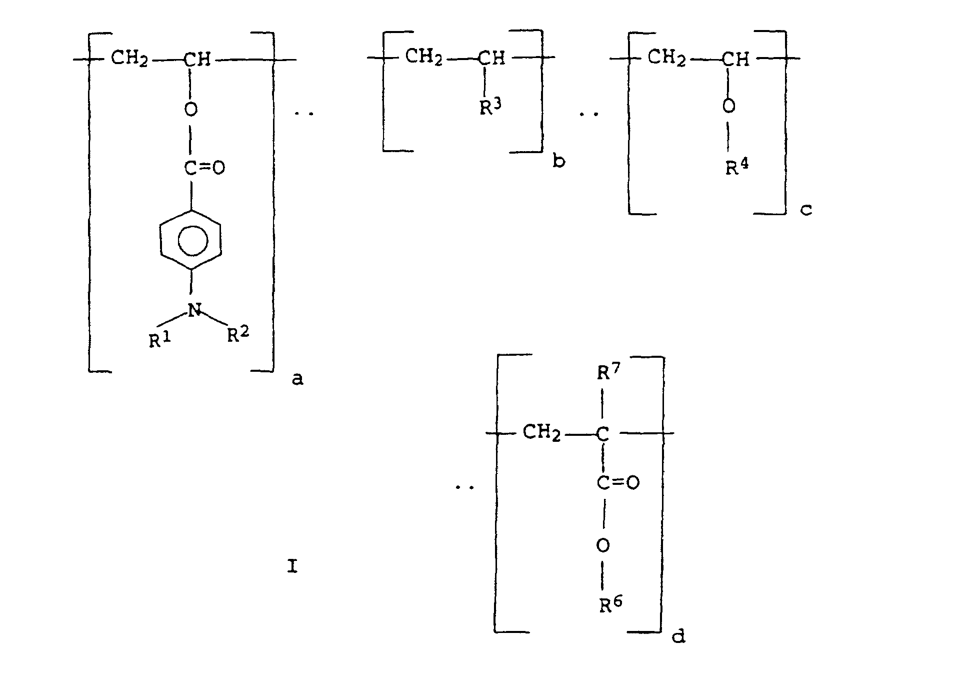 wobei
die Reihenfolge der Einheiten beliebig ist, die Summe aus a+b+c+d = 100 ist und
a für einen Wert von 5 bis 95 steht,
b für einen Wert von 0 bis 70 steht,
c für einen Wert von 5 bis 95 steht,
d für einen Wert von 0 bis 70 steht
R¹, R² unabhängig voneinander für H, C₁-C₈-Alkyl
R³ für 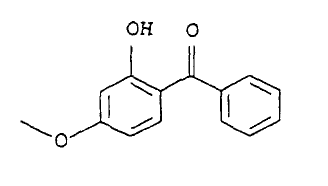 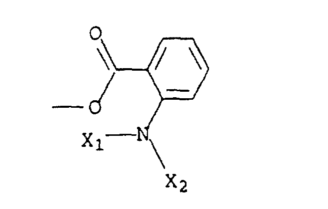
X¹, X² voneinander unabhängig für H, C₁ - C₈-Alkyl, 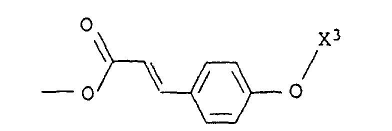
X³ für H, C₁ - C₈-Alkyl, 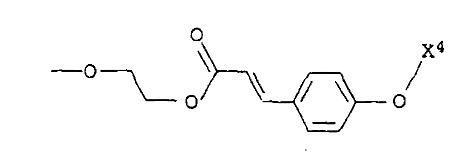
X⁴ für H, C₁ - C₈-Alkyl, 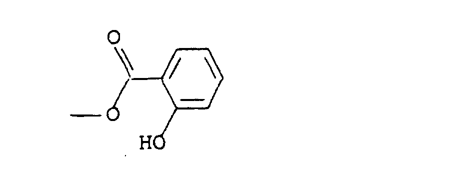 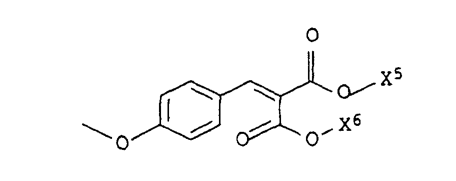
X⁵, X⁶ voneinander unabhängig für H, C₁ - C₈-Alkyl, 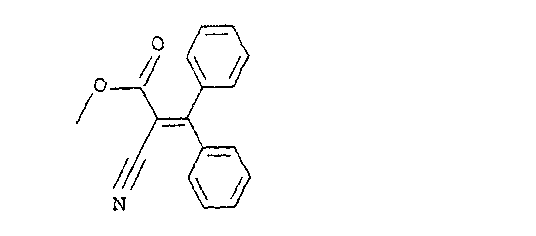 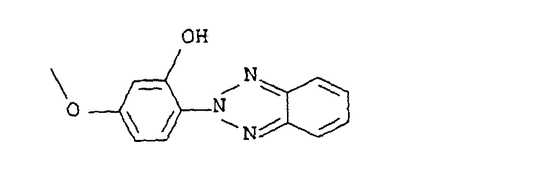 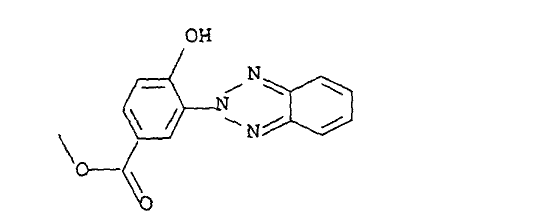 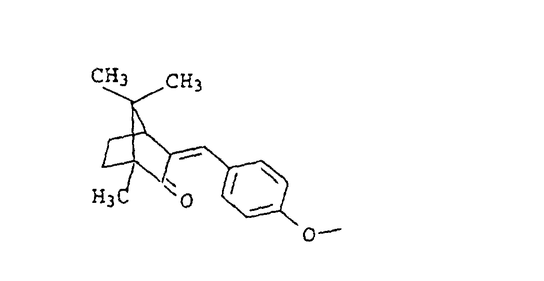 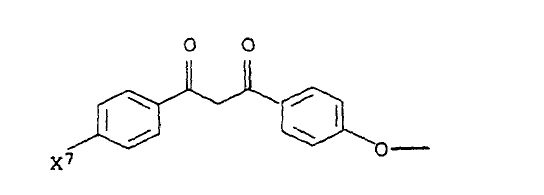
X⁷ für H, C₁- bis C₈-Alkyl;
R⁴ -(CO)ₙ-R⁵,
R⁵ für C₁-C₂₈-Alkyl,
n für 0 oder 1,
R⁶ für C₁-C₂₂-Alkyl,
R⁷, R⁸ unabhängig voneinander für H oder CH₃
steht.

2. Polymere nach Anspruch 1, wobei
R⁴ für -CO-R⁹,
R⁹ für ein verzweigtes Alkyl mit 6 bis 12 C-Atomen steht.

3. Polymere nach Anspruch 2, wobei R⁴ für 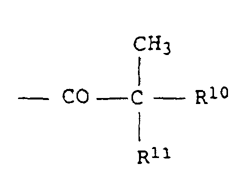
R¹⁰, R¹¹ für Alkyl mit der Maßgabe, daß R¹⁰ + R¹¹ 6 bis 7 C-Atome umfaßt.

4. Polymere nach Anspruch 1 bis 3, wobei
a für einen Wert von 15 bis 50,
b für einen Wert von 0 bis 70,
c für einen Wert von 30 bis 80,
d für einen Wert von 0 bis 70,
R⁶ für C₄- bis C₁₈-Alkyl steht.

5. Verwendung von polymeren UV-Absorbern gemäß Anspruch 1 bis 4 für kosmetische und pharmazeutische Zusammensetzungen.

6. Kosmetische oder pharmazeutische Zusammensetzungen, enthaltend neben üblichen Hilfsstoffen als wirksamen Bestandteil polymere UV-Absorber gemäß Anspruch 1 bis 4.

7. Verfahren zur Herstellung der Polymere gemäß Anspruch 1 bis 4, indem man die Polymerisation in einem kosmetischen Öl oder einem Ester aus langkettigem Fettalkohol durchführt.

## Claims

1. A polymeric UV absorber based on the units shown in the schematized formula I 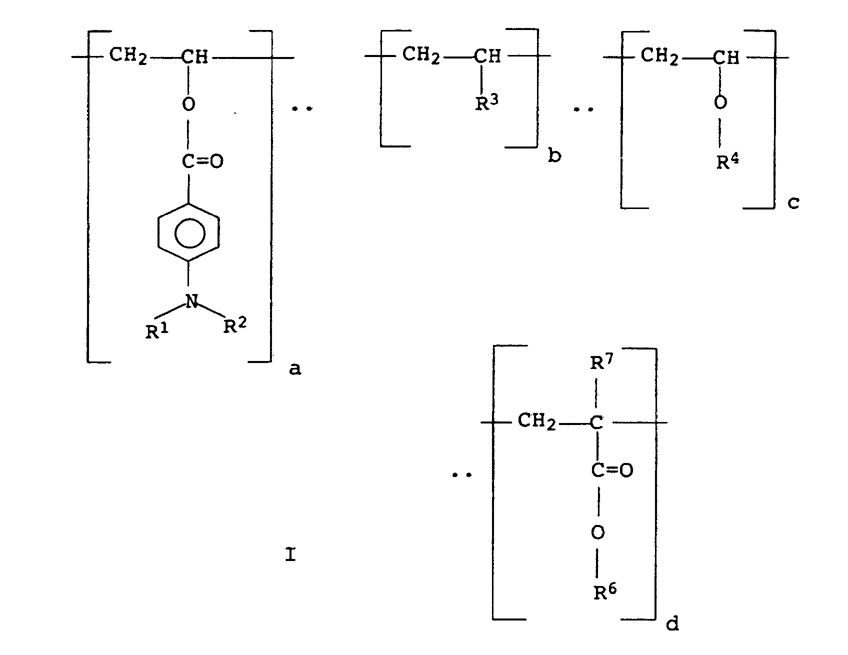 where
the sequence of the units is arbitrary, the sum of a+b+c+d = 100 and
a is a value from 5 to 95,
b is a value from 0 to 70,
c is a value from 5 to 95,
d is a value from 0 to 70
R¹,R² independently of one another are H, C₁-C₈-alkyl
R³ is 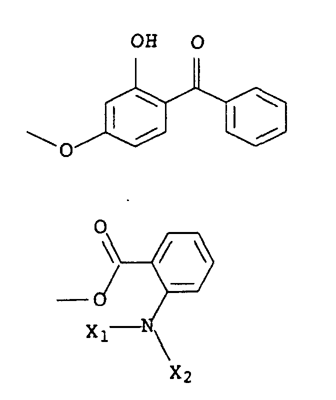
X¹, X² independently of one another are H, C₁-C₈-alkyl, 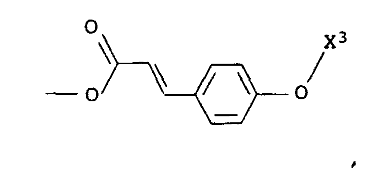
X³ is H, C₁-C₈-alkyl, 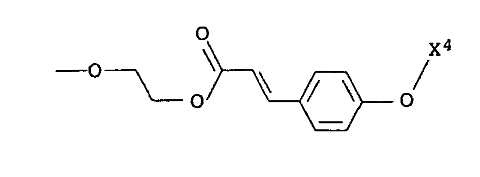
X⁴ is H, C₁-C₈-alkyl, 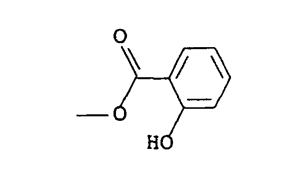 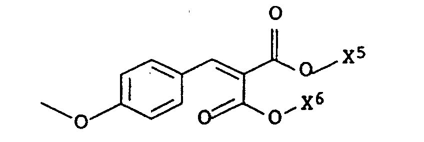
X⁵, X⁶ independently of one another are H, C₁-C₈-alkyl, 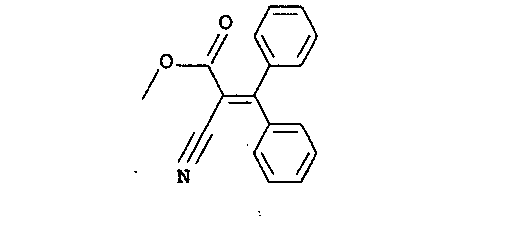 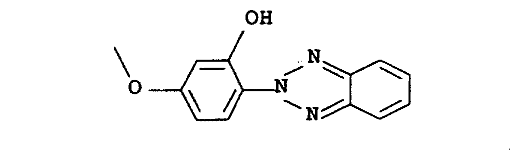 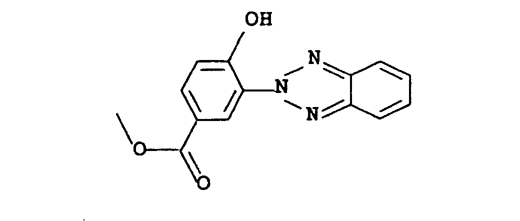 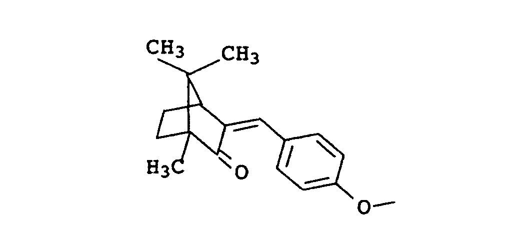 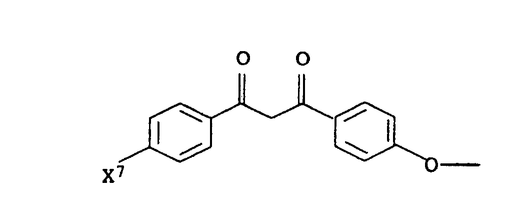
X⁷ is H, C₁- to C₈-alkyl;
R⁴ is -(CO)ₙ-R⁵,
R⁵ is C₁-C₂₈-alkyl,
n is 0 or 1,
R⁶ is C₁-C₂₂-alkyl,
R⁷, R⁸ independently of one another are H or CH₃.

2. A polymer as claimed in claim 1, where
R⁴ is -CO-R⁹,
R⁹ is a branched alkyl having 6 to 12 C atoms.

3. A polymer as claimed in claim 2, where R⁴ is 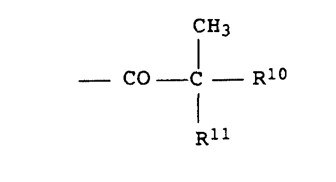
R¹⁰, R¹¹ are alkyl, with the proviso that R¹⁰ + R¹¹ comprises 6 to 7 C atoms.

4. A polymer as claimed in claims 1 to 3, where
a is a value from 15 to 50,
b is a value from 0 to 70,
c is a value from 30 to 80,
d is a value from 0 to 70,
R⁶ is C₄- to C₁₈-alkyl.

5. The use of polymeric UV absorbers as claimed in claims 1 to 4 for cosmetic and pharmaceutical compositions.

6. A cosmetic or pharmaceutical composition comprising, in addition to customary auxiliaries, a polymeric UV absorber as claimed in claims 1 to 4 as an active constituent.

7. A process for preparing the polymers as claimed in claims 1 to 4 by carrying out the polymerization in a cosmetic oil or an ester of long-chain fatty alcohol.

## Revendications

1. Polymères absorbant les UV, à base des motifs de la formule schématisée 1 : 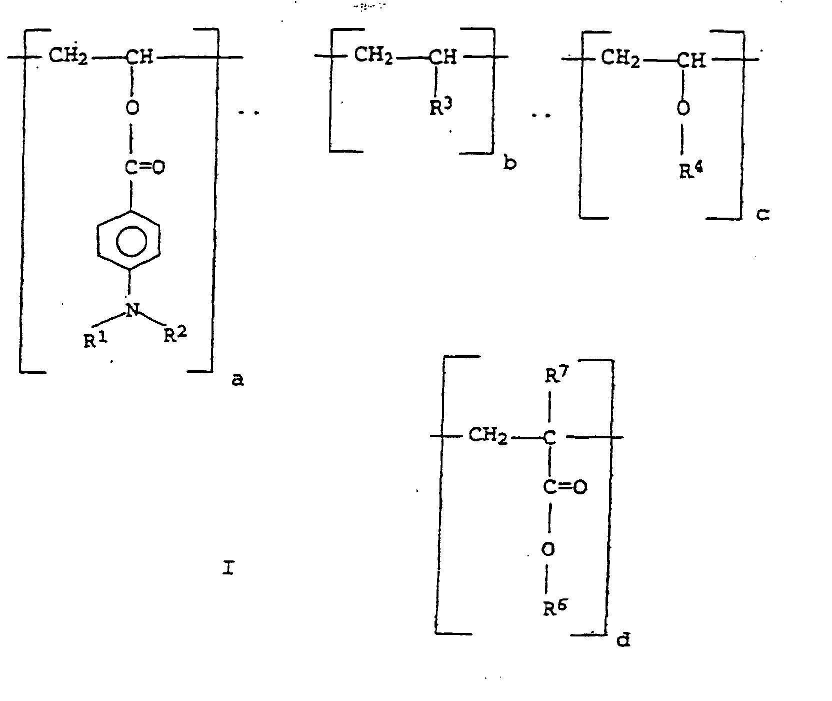 dans lesquels l'ordre des motifs est quelconque, la somme a+b+c+d est égale à 100 et
a est un n ombre allant de 5 à 95,
b est un nombre allant de 0 à 70,
c est un nombre allant de 5 à 95,
d est un nombre allant de 0 à 70,
R¹, R² représentent chacun, indépendamment l'un de l'autre, H, un groupe alkyle en C₁-C₈,
R³ représente 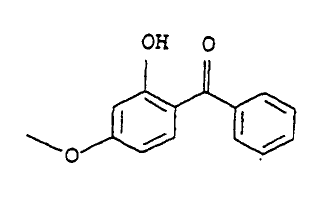 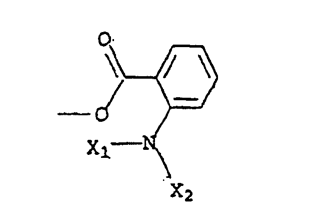
X¹, X² représentent chacun, indépendamment l'un de l'autre, H, un groupe alkyle en C₁-C₈, 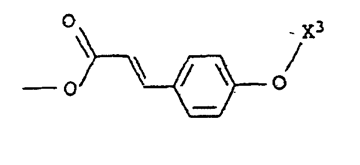
X³ représente H, un groupe alkyle en C₁-C₈, 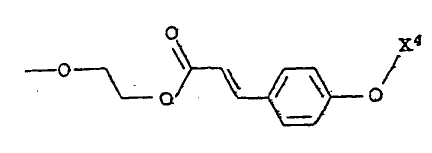
X⁴ représente H, un groupe alkyle en C₁-C₈, 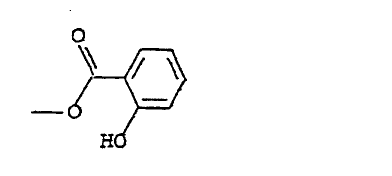 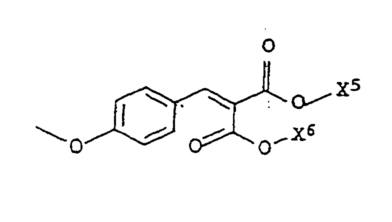
X⁵, X⁶ représentent chacun, indépendamment l'un de l'autre, H, un groupe alkyle en C₁-C₈ 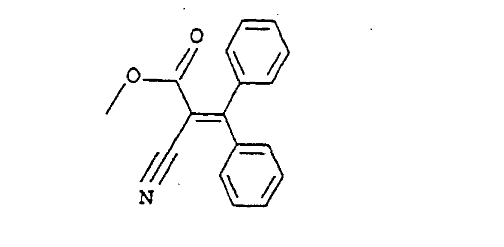 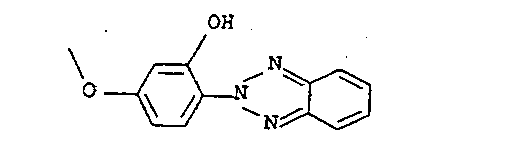 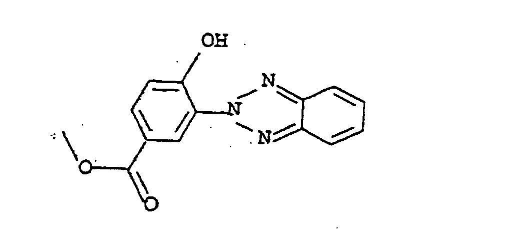 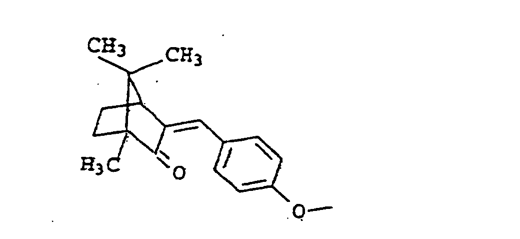 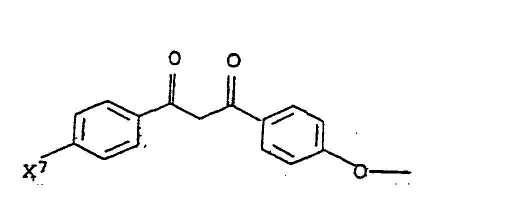
X⁷ représente H, un groupe alkyle en C₁-C₈,
R⁴ représente -(CO)ₙ-R⁵,
R⁸ représente un groupe alkyle en C₁-C₂₈,
n est égal à 0 ou 1,
R⁶ représente un groupe alkyle en C₁-C₂₂,
R⁷, R⁸ représentent chacun, indépendamment l'un de l'autre H ou CH₃.

2. Polymères selon la revendication 1 pour lesquels
R⁴ représente -CO-R⁹,
R⁹ représente un groupe alkyle à chaîne ramifiée en C₆-C₁₂.

3. Polymères selon la revendication 2, pour lesquels R⁴ représente 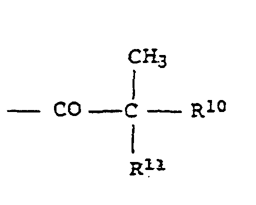 R¹⁰, R¹¹ représentent des groupes alkyle, sous réserve que R¹⁰ + R¹¹ contiennent au total de 6 à 7 atomes de carbone.

4. Polymères selon les revendications 1 à 3, pour lesquels
a est un nombre allant de 15 à 50,
b est un nombre allant de 0 à 70,
c est un nombre allant de 30 à 80,
d est un nombre allant de 0 à 70,
R⁶ représente un groupe alkyle en C₄-C₁₈.

5. Utilisation des absorbeurs d'UV polymères selon les revendications 1 à 4 dans des compositions cosmétiques et pharmaceutiques.

6. Compositions cosmétiques ou pharmaceutiques contenant, avec des produits auxiliaires usuels et en tant que constituants actifs, des absorbeurs d'UV polymères selon les revendications 1 à 4.

7. Procédé pour la préparation des polymères selon les revendications 1 à 4, selon lequel on procède à la polymérisation dans une huile cosmétique ou dans un ester d'alcool gras à longue chaîne.
